# EUROPEAN PATENT APPLICATION

(11) **EP 4 644 565 A1**
(43) Date of publication of application: **05.11.2025**
(21) Application number: 22969635.6
(22) Date of filing: 29.12.2022
(51) Int. Cl.: C12Q 1/68

(54) **SEQUENCING METHOD**

(71) Applicant: BGI Shenzhen, Shenzhen, Guangdong 518083 (CN); BGI Shenzhen Co., Ltd, Shenzhen, Guangdong 518083 (CN)
(72) Inventor: JI, Zhouxiang, Shenzhen, Guangdong 518083 (CN); LI, Yuxiang, Shenzhen, Guangdong 518083 (CN); WANG, Hao, Shenzhen, Guangdong 518083 (CN); ZENG, Tao, Shenzhen, Guangdong 518083 (CN); CHEN, Junyi, Shenzhen, Guangdong 518083 (CN); DONG, Yuliang, Shenzhen, Guangdong 518083 (CN); ZHANG, Wenwei, Shenzhen, Guangdong 518083 (CN); XU, Xun, Shenzhen, Guangdong 518083 (CN)
(74) Representative: Weickmann & Weickmann PartmbB
(86) International application number: PCT/CN2022/143120
(87) International publication number: WO 2024/138484

(57) **Abstract**

A nanopore sequencing method using a single-stranded specific nuclease, relating to the field of sequencing. The method can improve the sequencing flux, improve the capture efficiency of a target sequencing object, increase the number of holes (channels), increase an average read length of the target sequencing object, and/or improve the porin gating frequency.

## Description

### Technical Field

The present invention relates to the field of sequencing. Specifically, the present invention provides a nanopore sequencing method using a single-strand-specific nuclease.

### Background Art

Nanopore sequencing technology is an emerging single-molecule sequencing technology in recent years. Since it does not require amplification, with the advantages of simple operation, portability, ultra-long read length, low cost, and real-time monitoring, nanopore sequencing technology plays an important role in disease detection and genomic research. The most widely used commercial nanopore sequencing platform is the MinION nanopore meter developed by Oxford Nanopore Technologies.

The sequencing method adopted by the MinION nanopore meter is based on the speed control of helicase. Specifically, after the sequencing library is captured by the nanopore protein, the double-stranded DNA is unwound into two single-stranded DNAs under the action of the helicase, and one of the single-stranded DNA passes through the nanopore under the action of electric field force. The sequencing scheme based on the speed control of helicase can reach a sequencing speed of 500nt/s, with a relatively simple process of library construction, which brings about great advantages. However, as the sequencing time increases, problems such as reduced throughput and reduced capture rate often occur, thereby affecting the sequencing throughput and quality.

Therefore, an improved nanopore sequencing method is still in need to improve sequencing throughput and quality.

### Contents of the present invention

The inventors of the present application discovered through experiments that the remaining library loaded on the flow cell can cause "clogging" of the nanopore channel due to long-term sequencing, which is one of the reasons for the decrease in sequencing throughput. The remaining library includes the single-stranded DNA remaining after sequencing and a small amount of double-stranded DNA that has not been sequenced. Therefore, based on the experiences and experimental results, the inventors determined that the sequencing throughput and the capture rate of target sequence to be sequenced can be improved by eliminating the single-stranded DNA remaining after sequencing, and thus the following invention is provided.

In one aspect, the present invention provides a method for nucleic acid sequencing, which comprises performing nanopore sequencing on a polynucleotide to be tested under a condition comprising a single-strand-specific nuclease.

In certain embodiments, the condition comprising the single-strand-specific nuclease comprises adding the single-strand-specific nuclease to the sequencing system.

### Single-strand-specific nuclease

Single-strand-specific nuclease refers to a nuclease that can selectively act on single-stranded nucleic acids and single-stranded regions in double-stranded nucleic acids. Such nucleases are known to those skilled in the art, see, for example, Microbiology Reviews, 26(5), 2003, 457-491.

In some embodiments, the single-strand-specific nuclease is selected from an endonuclease or an exonuclease. In some embodiments, the single-strand-specific nuclease is selected from nuclease P1, nuclease T5, nuclease RecJ_{f}, nuclease HL.exo, nuclease Lambda, nuclease MBN, nuclease T7, nuclease S1, nuclease BAL 31, nuclease Mung bean, or any combination thereof.

In some embodiments, the single-strand-specific nuclease is selected from nuclease P1, nuclease T5, nuclease RecJ_{f}, nuclease HL.exo, or any combination thereof; for example, selected from nuclease P1, nuclease T5, nuclease HL.exo, or any combination thereof; for example, selected from nuclease P1, nuclease T5, nuclease HL.exo, or any combination thereof; for example, selected from nuclease P1, nuclease T5, or any combination thereof; for example, selected from nuclease P1.

In some embodiments, the single-strand-specific nuclease is capable of working in a salt-containing reaction system (e.g., a sequencing system). In some embodiments, the salt concentration of the sequencing system is at least 0.001M, at least 0.005M, at least 0.01M, at least 0.05M, at least 0.1M, at least 0.2M, or at least 0.3M. In some embodiments, the salt concentration of the sequencing system is 0.001-1M, such as 0.01-1M, 0.05-1M, 0.1-1M, 0.2-1M, 0.3-1M, 0.1-0.5M, 0.2-0.5M, or 0.3-0.5M.

In some embodiments, the concentration of the single-strand-specific nuclease is at least 0.001M, such as 0.001-1M.

In some embodiments, the concentration of nuclease P1 is 0.001-1M, such as 0.001-0.8M. In some embodiments, the concentration of nuclease T5 is 0.001-0.8M, such as 0.005-0.5M. In some embodiments, the concentration of nuclease RecJ_{f} is 0.001-0.5 M, such as 0.005-0.2 M. In some embodiments, the concentration of nuclease HL.exo is 0.001-0.8 M, such as 0.01-0.3M.

In some embodiments, the concentration of the single-strand-specific nuclease is at least 0.01 U/µL, such as at least 0.02 U/µL, at least 0.03 U/µL, at least 0.04 U/µL, at least 0.05 U/µL, at least 0.06 U/µL, at least 0.08 U/µL, at least 0.1 U/µL. In certain embodiments, the concentration of the single-strand-specific nuclease is at most 100 U/µL, at most 80 U/µL, at most 50 U/µL, at most 20 U/µL, at most 10 U/µL, at most 8 U/µL, at most 5 U/µL, at most 2 U/µL, at most 1 U/µL, at most 0.8 U/µL, at most 0.5 U/µL. In certain embodiments, the concentration of the single-strand-specific nuclease is 0.01-100 U/µL, for example, 0.01-50 U/µL, 0.01-20 U/µL, 0.01-10 U/µL, 0.01-8 U/µL, 0.01-5 U/µL, 0.01-2 U/µL, 0.01-1 U/µL, 0.01-0.8 U/µL, 0.01-0.5 U/µL, 0.02-10 U/µL, 0.02-8 U/µL, 0.02-5 U/µL, 0.02-2 U/µL, 0.02-1 U/µL, 0.02-0.8 U/µL, 0.02-0.5 U/µL, 0.03-10 U/µL, 0.03-8 U/µL, 0.03-5 U/µL, 0.03-2 U/µL, 0.03-1 U/µL, 0.03-0.8 U/µL, 0.03-0.5 U/µL.

### Nanopore sequencing

Nanopore sequencing refers to a method of determining the sequence of a polynucleotide with the aid of nanopore. Such methods are well known to those skilled in the art. The method disclosed herein is not limited to any specific nanopore sequencing method, system or device.

In certain embodiments, the nanopore sequencing comprises:
- providing a condition in a sequencing system comprising a polynucleotide to be tested and a sequencing solution so as to allow a single-stranded analyte of the polynucleotide to be tested to enter the nanopore; and
- obtaining one or more measurement values while moving the single-stranded analyte with respect to the nanopore, wherein the measurement value can be used to represent the sequence information of the polynucleotide to be tested.

In certain embodiments, the condition allowing the single-stranded analyte of the polynucleotide to be tested to enter the nanopore comprises: contacting the polynucleotide to be tested with a polynucleotide binding protein and a nanopore in the sequencing solution, wherein the polynucleotide binding protein controls the movement of the single-stranded analyte of the polynucleotide to be tested through the nanopore.

The polynucleotide to be tested may be contacted with the polynucleotide binding protein and the nanopore in any order. In certain embodiments, the polynucleotide first forms a complex with the polynucleotide binding protein. When a voltage is applied across the nanopore, the polynucleotide/polynucleotide binding protein complex forms a complex with the pore and controls the movement of the polynucleotide through the pore.

The polynucleotide binding protein described herein may be any protein capable of binding to a polynucleotide and controlling its movement through the pore. Such proteins are well known to those skilled in the art, such as polymerases, helicases, translocases, topoisomerases. In certain embodiments, the polynucleotide binding protein is selected from a nucleic acid helicase, such as a DNA helicase or an RNA helicase. In certain embodiments, the polynucleotide binding protein is selected from Dda, UvrD, Rep, RecQ, PcrA, eIF4A, NS3, Rep, gp41 or T7gp4.

In certain embodiments, the measurement value is an ionic current through the nanopore. Appropriate conditions for measuring ionic currents through transmembrane protein pores are known in the art, and may be selected by those skilled in the art based on practical requirements. In certain embodiments, the method is typically performed with a voltage applied across the membrane and the pore. In certain embodiments, the voltage used is typically +5V to -5V, such as +4V to -4V, +3V to -3V, +2V to -2V, -600mV to +600mV, or -400mV to +400mV. In certain embodiments, the voltage used is in the range of 100mV to 240mV, such as 120mV to 220mV.

In certain embodiments, the condition allowing the single-stranded analyte of the polynucleotide to be tested to enter the nanopore also comprises: applying a potential difference across the nanopore to allow the single-stranded analyte to enter the nanopore.

In certain embodiments, the sequencing system comprises a sequencing solution, and the sequencing solution comprises a charge carrier, such as a salt (e.g., potassium chloride (KCl), sodium chloride (NaCl), cesium chloride (CsCl), or a mixture of potassium ferrocyanide and potassium ferricyanide). In some embodiments, the salt concentration is at least 0.001M, at least 0.005M, at least 0.01M, at least 0.05M, at least 0.1M, at least 0.2M, or at least 0.3M. In some embodiments, the salt concentration is 0.001-1M, such as 0.01-1M, 0.05-1M, 0.1-1M, 0.2-1M, 0.3-1M, 0.1-0.5M, 0.2-0.5M, or 0.3-0.5M.

In some embodiments, the sequencing solution further comprises a buffer, such as a HEPES buffer, a phosphate buffer, or a Tris-HCl buffer. In some embodiments, the concentration of the buffer is 10-100mM, such as 10-90mM, 10-80mM, 10-70mM, 10-60mM, 10-50mM, 20-50mM, 20-40mM or 20-30mM.

In some embodiments, any step in the method using a polynucleotide binding protein is typically performed in the presence of a free nucleotide and an enzyme cofactor that promote the action of the polynucleotide binding protein. Therefore, in some embodiments, the sequencing solution also comprises a free nucleotide and/or an enzyme cofactor.

In some embodiments, the free nucleotide is selected from AMP, TMP, GMP, CMP, UMP, dAMP, dTMP, dGMP or dCMP. In some embodiments, the free nucleotide is adenosine triphosphate (ATP). In some embodiments, the concentration of the free nucleotides is 10-100mM, such as 10-90mM, 10-80mM, 10-70mM, 10-60mM, 10-50mM, 20-50mM, or 20-40mM.

In some embodiments, the enzyme cofactor is selected from divalent metal cations, such as Mg²⁺, Mn²⁺, Ca²⁺ or Co²⁺. In some embodiments, the enzyme cofactor is Mg²⁺. In some embodiments, the concentration of the enzyme cofactor is 10-100mM, such as 10-90mM, 10-80mM, 10-70mM, 10-60mM, 10-50mM, 20-50mM, 20-40mM or 20-30mM.

In some embodiments, the sequencing solution comprises potassium chloride (KCl), HEPES buffer, ATP and Mg²⁺ (e.g., MgCl₂). For example, the concentration of KCl is at least 0.001M (e.g., at least 0.005M, at least 0.01M, at least 0.05M, at least 0.1M, at least 0.2M, or at least 0.3M; for example, 0.001-1M, for example, 0.01-1M, 0.05-1M, 0.1-1M, 0.2-1M, 0.3-1M, 0.1-0.5M, 0.2-0.5M, or 0.3-0.5M; for example, 150mM, 300mM or 470mM). For example, the concentration of HEPES buffer is 10-100mM (e.g., 10-90mM, 10-80mM, 10-70mM, 10-60mM, 10-50mM, 20-50mM, 20-40mM or 20-30mM, for example, 25nM). For example, the concentration of ATP is 10-100mM (e.g., 10-90mM, 10-80mM, 10-70mM, 10-60mM, 10-50mM, 20-50mM, or 20-40mM; for example, 30mM). For example, the concentration of Mg²⁺ (e.g., MgCl₂) is 10-100mM (e.g., 10-90mM, 10-80mM, 10-70mM, 10-60mM, 10-50mM, 20-50mM, 20-40mM or 20-30mM; for example, 25mM).

Those skilled in the art understand that when the single-strand-specific nuclease is a metal-dependent nuclease, the corresponding reaction solution (sequencing system) will contain corresponding metal ions (e.g., zinc ions or sodium ions). For example, nuclease P1 is Zn ion-dependent type, therefore the sequencing solution may also contain zinc ions when nuclease P1 is used as a single-strand-specific nuclease.

In some embodiments, the polynucleotide to be tested is a double-stranded polynucleotide, such as double-stranded DNA.

In some embodiments, the double-stranded polynucleotide comprises at least one single-stranded overhang (e.g., 5'-end overhang and/or 3'-end overhang), and the single-stranded overhang contains a leader sequence, and the leader sequence leads the nucleic acid strand attached thereto to enter into the nanopore.

In some embodiments, the leader sequence contains a spacer region, and the spacer region is capable of stalling the helicase. In some embodiments, the spacer region is composed of one or more (e.g., 2, 3, 4, 5 or 6, such as 4) iSp18. iSp18 refers to 18-atom hexa-ethyleneglycol spacer, which has the following structure:

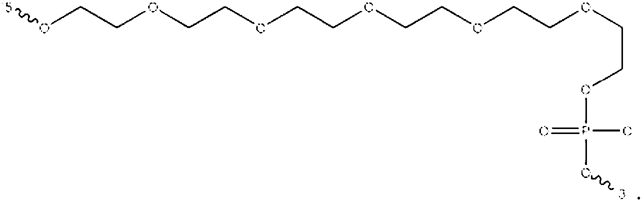

In some embodiments, the sequencing system further comprises one or more tethers, wherein the tether comprises a capture sequence having sequence complementarity with a portion of the polynucleotide to be tested, and the polynucleotide to be tested is coupled to the outer rim or vicinity of the nanopore by hybridization of the capture sequence with the complementary region in the polynucleotide to be tested.

In some embodiments, the tether further comprises a cholesterol molecule. Cholesterol molecule can be embedded in the membrane, thereby anchoring the polynucleotide to be tested complementary to the tether.

In some embodiments, the cholesterol molecule in the tether can be connected to the capture sequence through a spacer molecule to enhance the flexibility of the tether itself, which is beneficial for binding to the polynucleotide to be tested. In some embodiments, the spacer molecule is composed of one or more (e.g., 2, 3, 4, 5 or 6, such as 4) iSp18.

It is easy to understand that the tether is used to specifically or non-specifically enrich the polynucleotide to be tested in the vicinity of the pore. Therefore, those skilled in the art can design the length and number of the tether and the capture sequence (specific or non-specific capture sequence) according to the requirements, and the present application is not limited thereto.

In some embodiments, the nanopore is disposed in a membrane. The membrane typically separates two volumes of aqueous solution. The membrane resists the electric current flow between the volumes. The nanopore inserted into the membrane selectively allows ions to pass through the membrane, which can be recorded as an electrical signal detected by electrodes in two volumes of aqueous solution. Suitable membranes are well known in the art. The membrane is preferably an amphiphilic layer or a solid layer. The amphiphilic layer is a layer formed by amphiphilic molecules, which have both hydrophilic and lipophilic properties. The amphiphilic molecule can be synthetic (e.g., block copolymers) or naturally occurring (e.g., phospholipids). In some embodiments, the membrane is an amphiphilic layer, such as a lipid bilayer (e.g., a phospholipid bilayer) or a high molecular polymer membrane (e.g., di-block, tri-block).

In some embodiments, the nanopore is a transmembrane pore that allows hydrated ions driven by an applied potential to flow across or within the membrane. Transmembrane pores typically cross the entire membrane so that hydrated ions can flow from one side of the membrane to the other side of the membrane. Any transmembrane pore can be used in the present invention. The pore can be natural or artificial. Suitable pores include, but are not limited to, protein pores, polynucleotide pores, and solid-state pores.

In some embodiments, the nanopore is a transmembrane protein pore, which typically comprises a barrel or channel through which ions can flow. The subunits of the pore typically surround a central axis and contribute strands to a transmembrane barrel or channel.

In some embodiments, the nanopore is typically composed of several repeating subunits, such as at least 6, at least 7, at least 8, at least 9, at least 10, at least 11, at least 12, at least 13, at least 14, at least 15, or at least 16 subunits. In some embodiments, the pore is preferably a hexamer, heptamer, octamer or nonamer pore. In some embodiments, the pore can be a homo-oligomer or a hetero-oligomer.

In some embodiments, the nanopore is derived from or based on a bacteriophage (e.g., phi29, SPP1, T3, T4, T7) DNA packaging motor. For example, see Ji, Zhouxiang, and Peixuan Guo. "Channel from bacterial virus T7 DNA packaging motor for the differentiation of peptides composed of a mixture of acidic and basic amino acids." Biomaterials 214 (2019): 119222.

### Addition of single-strand-specific nuclease

The addition of a single-strand-specific nuclease to the sequencing system described herein can be implemented in any manner, and the present application is not limited thereto. For example, a single-strand-specific nuclease can be directly added to the original sequencing solution. For example, a fresh sequencing solution containing a single-strand-specific nuclease can also be prepared to replace the original sequencing solution. In addition, incubation can be optionally included after adding a single-strand-specific nuclease to the sequencing system.

In some embodiments, a single-strand-specific nuclease may be added to the sequencing system before the start of, during and/or after sequencing.

In some embodiments, a single-strand-specific nuclease is added to the sequencing system before the start of sequencing. In some embodiments, a single-strand-specific nuclease is added to the sequencing system before the start of sequencing followed by incubation (e.g., at 30°C) for, e.g., at least 2 min, at least 5 min, at least 8 min, at least 10 min, at least 15 min, at least 20 min, at least 30 min, e.g., 2-30 min, 5-30 min, 8-30 min, 10-30 min, 15-30 min, 20-30 min.

In some embodiments, the method comprises: providing a condition in the sequencing system with the addition of the single-strand-specific nuclease so as to allow the single-stranded analyte of the polynucleotide to be tested to enter the nanopore for sequencing.

In some embodiments, a single-strand-specific nuclease is added to the sequencing system during sequencing. In some embodiments, a single-strand-specific nuclease is added to the sequencing system during sequencing followed by incubation (e.g., 30°C) for, e.g., at least 2 min, at least 5 min, at least 8 min, at least 10 min, at least 15 min, at least 20 min, at least 30 min, e.g., 10-30 min, 15-30 min, 20-30 min.

In some embodiments, the method comprises:
- providing a condition in the sequencing system without addition of a single-strand-specific nuclease so as to allow the single-stranded analyte of the polynucleotide to be tested to enter the nanopore to start sequencing;
- adding the single-strand-specific nuclease to the sequencing system one or more times.

In some embodiments, incubation is optionally comprised after each addition of the single-strand-specific nuclease.

In some embodiments, the method comprises:
- providing a condition in the sequencing system without addition of the single-strand-specific nuclease so as to allow the single-stranded analyte of the polynucleotide to be tested to enter the nanopore to start sequencing;
- pausing sequencing and adding the single-strand-specific nuclease to the sequencing system (and optionally performing incubation);
- resuming sequencing.

In some embodiments, the steps of "pausing sequencing and adding the single-strand-specific nuclease to the sequencing system (and optionally performing incubation); resuming sequencing" are repeated one or more times until the sequencing is completed.

In some embodiments, a single-strand-specific nuclease is added to the sequencing system after the sequencing is completed. In some embodiments, a single-strand-specific nuclease is added to the sequencing system after the sequencing is completed, followed by incubation (e.g., at 30°C) for, e.g., at least 2min, at least 5min, at least 8min, at least 10min, at least 15min, at least 20min, at least 30min, e.g., 2-30min, 5-30min, 8-30min, 10-30min, 15-30min, 20-30min. In some embodiments, a washing step (e.g., using a sequencing solution) is further comprised after the incubation. In some embodiments, the method further comprises proceeding to a next round of sequencing. In some embodiments, after the sequencing is completed, the remaining single strand after the sequencing can be degraded by adding a nuclease working in the 5' to 3' direction, thereby releasing the binding site of the tether, and thus achieve the regeneration of the tether.

In some embodiments, the method comprises the following steps:
(1) providing a double-stranded polynucleotide to be tested that comprises a single-stranded overhang, in which the single-stranded overhang is linked with a polynucleotide binding protein;
(2) contacting the double-stranded polynucleotide to be tested of step (1) with a nanopore in a sequencing solution comprising a single-strand-specific nuclease, in which the polynucleotide binding protein separates the two strands of the double-stranded polynucleotide to provide a single-stranded polynucleotide (i.e., a single-stranded analyte), and controls the movement of the single-stranded analyte through the pore; and,
(3) obtaining one or more measurement values while moving the single-stranded analyte with respect to the nanopore, wherein the measurement values can be used to represent the sequence information of the polynucleotide to be tested.

In some embodiments, step (2) comprises: providing the double-stranded polynucleotide to be tested of step (1), the single-strand-specific nuclease and a tether in the sequencing solution and incubating, and then contacting the incubated solution with the nanopore.

In another aspect, the present invention provides a kit comprising a single-strand-specific nuclease.

In some embodiments, the kit is used for nanopore sequencing.

In some embodiments, the single-strand-specific nuclease is selected from an endonuclease or an exonuclease. In some embodiments, the single-strand-specific nuclease is selected from nuclease P1, nuclease T5, nuclease RecJ_{f}, nuclease HL.exo, nuclease Lambda, nuclease MBN, nuclease T7, nuclease S1, nuclease BAL 31, nuclease Mung bean, or any combination thereof. In some embodiments, the single-strand-specific nuclease is selected from nuclease P1, nuclease T5, nuclease RecJ_{f}, nuclease HL.exo, or any combination thereof; for example, selected from nuclease P1, nuclease T5, nuclease HL.exo, or any combination thereof; for example, selected from nuclease P1, nuclease T5, or any combination thereof; for example, selected from nuclease P1.

In some embodiments, the single-strand-specific nuclease is capable of working in a salt-containing reaction system (e.g., a sequencing system). In some embodiments, the salt concentration of the sequencing system is at least 0.001M, at least 0.005M, at least 0.01M, at least 0.05M, at least 0.1M, at least 0.2M, or at least 0.3M. In some embodiments, the salt concentration of the sequencing system is 0.001-1M, such as 0.01-1M, 0.05-1M, 0.1-1M, 0.2-1M, 0.3-1M, 0.1-0.5M, 0.2-0.5M, or 0.3-0.5M.

In some embodiments, the concentration of the single-strand-specific nuclease is at least 0.001M, such as 0.001-1M.

In some embodiments, the concentration of nuclease P1 is 0.001-1M, such as 0.001-0.8M. In some embodiments, the concentration of nuclease T5 is 0.001-0.8M, such as 0.005-0.5M. In some embodiments, the concentration of nuclease RecJ_{f} is 0.001-0.5 M, such as 0.005-0.2 M. In some embodiments, the concentration of nuclease HL.exo is 0.001-0.8 M, such as 0.01-0.3 M.

In some embodiments, the concentration of the single-strand-specific nuclease is at least 0.01 U/µL, such as at least 0.02 U/µL, at least 0.03 U/µL, at least 0.04 U/µL, at least 0.05 U/µL, at least 0.06 U/µL, at least 0.08 U/µL, at least 0.1 U/µL. In some embodiments, the concentration of the single-strand-specific nuclease is 0.01-100 U/µL, such as 0.01-50 U/µL, 0.01-20 U/µL, 0.01-10 U/µL, 0.01-8 U/µL, 0.01-5 U/µL, 0.01-2 U/µL, 0.01-1 U/µL, 0.02-10 U/µL, 0.02-8 U/µL, 0.02-5 U/µL, 0.02-2 U/µL, 0.02-1 U/µL, 0.03-10 U/µL, 0.03-8 U/µL, 0.03-5 U/µL, 0.03-2 U/µL, 0.03-1 U/µL.

In some embodiments, the kit further comprises a reagent for nanopore sequencing.

In certain embodiments, the reagent for nanopore sequencing comprises one or more selected from the following: a nanopore or subunit thereof (e.g., transmembrane protein pore or subunit thereof), a membrane, a polynucleotide binding protein, a sequencing solution, a tether.

In certain embodiments, the nanopore, membrane, polynucleotide binding protein, sequencing solution, tether are as defined in any of the above embodiments.

On the other hand, the present invention also provides the use of the kit for nanopore sequencing.

On the other hand, the present invention also provides the use of single-strand-specific nuclease for nanopore sequencing. In certain embodiments, the single-strand-specific nuclease is as defined in any of the above embodiments.

### Beneficial effects of the invention

The inventors of the present application have discovered for the first time that the residual single-stranded DNA in sequencing is one of the main reasons for the decrease in sequencing throughput, thereby providing a method for using single-strand-specific nuclease to improve the throughput and capture rate of nanopore sequencing for the first time. The method of the present application can improve sequencing throughput, improve the capture efficiency of target sequencing analyte, increase the number of pores (channels), increase the average read length of target sequencing analyte, and reduce the gating frequency of pore protein. The method of the present application demonstrates broad application prospects.

The embodiments of the present invention will be described in detail below in conjunction with the accompanying drawings and examples, but those skilled in the art will understand that the following drawings and examples are only used to illustrate the present invention, rather than to limit the scope of the present invention. Based on the following detailed description of the drawings and preferred embodiments, the various objects and advantages of the present invention will become apparent to those skilled in the art.

### Brief Description of the Drawings

FIG. 1 shows the activity test results of different nucleases in degrading single-stranded DNA according to Example 2 of the present invention.
FIG. 2 shows the activity test results of different nucleases in degrading single-stranded DNA in low-salt solution according to Example 2 of the present invention.
FIG. 3 shows the activity test results of nuclease P1 in degrading single-stranded DNA in solutions of different salt concentrations according to Example 2 of the present invention.
FIG. 4 shows the activity test results of nuclease P1 in degrading double-stranded DNA in solutions of different salt concentrations according to Example 2 of the present invention.
FIG. 5 shows the time-dependent activity test results of nuclease P1 in degrading single-stranded DNA in high-salt solution according to Example 2 of the present invention.
FIG. 6 shows the signal diagram obtained by sequencing on a nanopore sequencing platform according to Example 3 of the present invention. FIG. 6(1) shows the signal diagram at the beginning of sequencing, and FIG. 6(2) shows the signal diagram after 16 hours of sequencing.
FIG. 7 shows the signal diagram obtained by sequencing on a nanopore sequencing platform according to Example 4 of the present invention. FIG. 7(1) shows the signal diagram obtained after 16 hours of sequencing and before addition of nuclease P1, and FIG. 7(2) shows the signal diagram obtained after continued sequencing for 30 minutes following addition of nuclease P1 for digestion and washing.
FIG. 8 shows the signal diagram obtained by sequencing on a nanopore sequencing platform according to Example 4 of the present invention. FIG. 8(1) shows the signal diagram obtained after 16 hours of sequencing and before addition of nuclease composition, and FIG. 8(2) shows the signal diagram obtained after continued sequencing for 30 minutes following addition of P1 and T5 nuclease composition for digestion and washing.
FIG. 9 shows the signal diagram obtained by sequencing on a nanopore sequencing platform according to Example 4 of the present invention. FIG. 9(1) shows the signal diagram obtained after 16 hours of sequencing and before addition of sequencing solution 3 for washing, and FIG. 9(2) shows the signal diagram obtained after continued sequencing for 30 minutes following addition of sequencing solution 3 for washing.
FIG. 10 shows the signal diagram obtained by sequencing on a nanopore sequencing platform according to Example 5 of the present invention. FIG. 10(1) shows the sequencing signal diagram of the control group (incubated without enzyme), and FIG. 10(2) shows the sequencing signal diagram of the experimental group (incubated with nuclease P1).
FIG. 11 shows the effect of different concentrations of nuclease P1 on the reads obtained in a single DNA channel according to Example 6 of the present invention.
FIG. 12 shows the effect of different concentrations of nuclease P1 on the average DNA read length according to Example 6 of the present invention.
FIG. 13 shows the effect of different concentrations of nuclease P1 on sequencing speed according to Example 6 of the present invention.
FIG. 14 shows the effect of nuclease P1 on the gating event of pore protein during sequencing according to Example 7 of the present invention. FIG. 14(1) shows the gating frequency diagram of the control group without addition of nuclease P1, and FIG. 14(2) shows the gating frequency diagram of the experimental group with addition of nuclease P1.
FIG. 15 shows the effect of different time points of adding nuclease P1 on the reads obtained in a single DNA channel according to Example 8 of the present invention.
FIG. 16 shows the effect of different time points of adding nuclease P1 on the average DNA read length according to Example 8 of the present invention.
FIG. 17 shows the effect of different time points of adding nuclease P1 on the effective time of DNA sequencing according to Example 8 of the present invention.
FIG. 18 shows the signal diagrams before (1) and after (2) the addition of nuclease HL-Exol according to Example 9 of the present invention.

### EXAMPLES

### Sequence information

The description of the sequences involved in the present application is provided in the following table.

| SEQ ID NO | Description | Sequence |
|---|---|---|
| 1 | Sequence 1 | TTTTTTTTTTTTTTTTTTTTGGTTGTTTCTGTTGGTGCT GATATTGC-FAM |
| 2 | Sequence 2 | FAM-CGTTATAGTCGTGGTTGTCTTTGTTGGTTTTTTT TTTTTTTTTTTTTTTTTTTTTTTTTTTTTTTT |
| 3 | Sequence 3-sense strand | FAM-GCAATATCAGCACCAACAGAAACAACC |
| 4 | Sequence 3-antisense strand | GGTTGTTTCTGTTGGTGCTGATATTGC |
| 5 | Tether sequence | Cholesterol-YYYYTTGACCGCTCGCCTC (Y=iSp18) |
| 6 | Sequence 4 | TTTTTTTTTTTTTTTTTTTTGGTTGGTGTGGTTGG |

The present invention will now be described with reference to the following examples which are intended to illustrate the present invention (but not to limit the present invention).

Unless otherwise specified, the molecular biology experimental methods and immunoassays used in the present invention are basically carried out with reference to the methods described in J. Sambrook et al., Molecular Cloning: A Laboratory Manual, 2^{nd} edition, Cold Spring Harbor Laboratory Press, 1989, and F. M. Ausubel et al., Short protocols in molecular biology, 3^{rd} edition, John Wiley & Sons, Inc., 1995; if the specific conditions are not specified in the examples, they are carried out according to conventional conditions or conditions recommended by the manufacturer. The reagents or instruments used without indicating the manufacturer are all conventional products that can be obtained commercially. Those skilled in the art know that the examples describe the present invention by way of example and are not intended to limit the scope of protection claimed by the present invention.

### Example 1. Preparation of sequencing solution

Sequencing solutions with different salt concentrations were prepared according to Table 1 for use in subsequent examples.

**Table 1. Formulations of sequencing solutions with different KCl concentrations**

| Solution No. | KCl (mM) | HEPES (mM) | ATP (mM) | MgCl₂ (mM) | Nuclease P1 (activity unit U/µL) | Nuclease T5 (activity unit U/µL) | Nuclease HL-Exol (activity unit U/µL) |
|---|---|---|---|---|---|---|---|
| Sequencing solution 1 | 150 | 25 | 30 | 25 | / | / | / |
| Sequencing solution 2 | 300 | 25 | 30 | 25 | / | / | / |
| Sequencing solution 3 | 470 | 25 | 30 | 25 | / | / | / |
| Sequencing solution 4 | 470 | 25 | 30 | 25 | 1 | / | / |
| Sequencing solution 5 | 470 | 25 | 30 | 25 | 0.333 | 0.033 | / |
| Sequencing solution 6 | 470 | 25 | 30 | 25 | 0.033 | / | / |
| Sequencing solution 7 | 470 | 25 | 30 | 25 | 0.067 | / | / |
| Sequencing solution 8 | 470 | 25 | 30 | 25 | 0.133 | / | / |
| Sequencing solution 9 | 300 | 25 | 30 | 25 | / | / | 0.034 |

| | | | | | | | |
|---|---|---|---|---|---|---|---|
| Note: For metal-dependent nucleases, e.g., nuclease P1 of Zn-dependent type, the nuclease P1 stock solution contained a certain amount of zinc ions and sodium ions according to its instructions for use, which were not listed in the table since their amounts were very low. | | | | | | | |

### Example 2. Detection of nuclease activity

### Reagents:

Nuclease P1 (NEB, M0660S, 100000 units/mL), mung bean nuclease MBN (NEB, M0250S, 10000 units/mL), nuclease Lambda (NEB, M0262, 5000 units/mL), nuclease T5 (NEB, M0663S, 10,000 units/mL), nuclease T7 (NEB, M0263S, 10,000 units/mL), nuclease RecJ_{f} (NEB, M0264S, 30000 units/mL), nuclease HL-Exol (HL.exo, ArcticZymes Technologies, 70100-201, 34.4 U/µL), single-stranded DNA sequence 1 (SEQ ID NO: 1, 100 µM, synthesized by BGI TECH SOLUTIONS (BEIJING LIUHE) CO., LIMITED), single-stranded DNA sequence 2 (SEQ ID NO: 2, 100 µM, synthesized by BGI TECH SOLUTIONS (BEIJING LIUHE) CO., LIMITED), double-stranded DNA sequence 3 (formed by annealing SEQ ID NO: 3 and SEQ ID NO: 4, and synthesized by BGI TECH SOLUTIONS (BEIJING LIUHE) CO., LIMITED).

### Experimental method:

1. The reaction solutions of the experimental group and the control group were prepared according to Table 2. The above single-stranded DNA (100 µM) was added to the solutions containing different enzymes, and prepared using the diluent provided with the nuclease reagent. Among them, the nucleases used were: P1, MBN, T5, Lambda and RecJ_{f}. Single-stranded DNA sequence 1 was used to test nuclease P1, MBN, T5, Lambda and RecJ_{f}, and single-stranded DNA sequence 2 was used to test nuclease HL.exo.

The prepared liquid was mixed evenly, incubated at 30 °C for 1 hour, and then subjected to 20% TBU polyacrylamide gel electrophoresis after the reaction.

**Table 2: Amounts of solution and enzyme added in enzyme activity detection experiment**

| Sequencing solution | DNA (µL) | Solution (µL) | Nuclease (µL) |
|---|---|---|---|
| Experimental group | 1 | 8 | 1 |
| Control group | 1 | 9 | 0 |

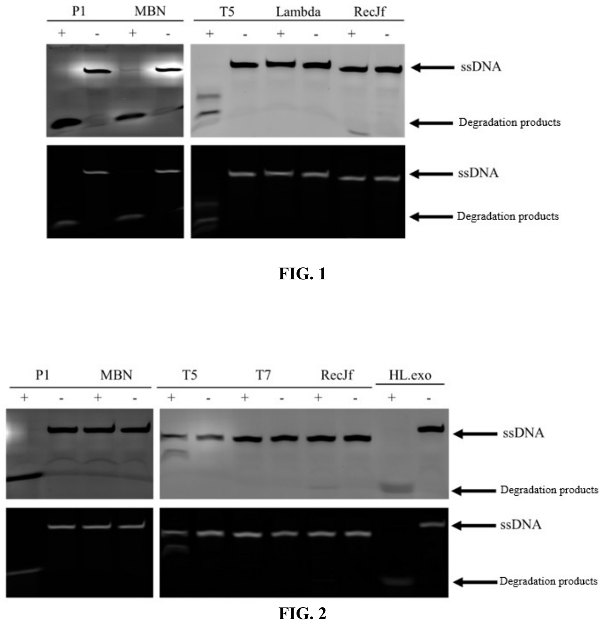

The results were shown in FIG. 1. It could be seen from the figure that, under the condition using the diluent provided with the nuclease reagent, the nucleases P1, MBN, T5 and RecJ_{f} in the experimental group all generated certain degradation products, indicating certain enzymatic activity under the experimental condition.

2. The diluent provided with the nuclease reagent in step (1) was replaced with sequencing solution 1. The nucleases used were: P1, MBN, T5, T7, RecJ_{f} and HL.exo. Correspondingly, the single-stranded DNA sequence 1 was used to test nucleases P1, MBN, T5 and RecJ_{f}, and the single-stranded DNA sequence 2 (100 µM) was used to test nuclease HL.exo. All other conditions remained unchanged, the test was performed again according to the above steps.

The results were shown in FIG. 2. It could be seen from the figure that, under the condition of 150 mM KCl, the nucleases P1, T5 and HL.exo of the experimental group all generated degradation products, indicating certain enzymatic activity under the experimental condition.

3. In the nanopore sequencing, a certain salt concentration that was generally greater than 0.3M KCL was usually required to ensure the signal-to-noise ratio of sequencing. Therefore, the diluent provided with the nuclease reagent in step (1) was replaced with the sequencing buffer of three different salt concentrations (0.15 M, 0.3 M, 0.47 M) as prepared in Example 1, and the enzyme activity of nuclease P1 was tested using the single-stranded DNA sequence 1 and the double-stranded DNA sequence 3. All other conditions remained unchanged, the test was performed again according to the above steps.

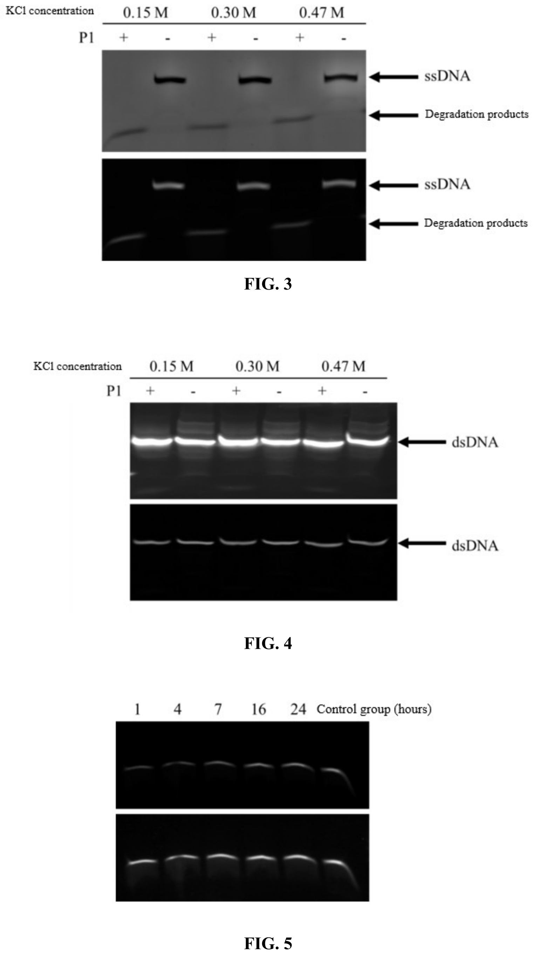

The results were shown in FIGS. 3 and 4. It could be seen from FIG. 3 that, under the conditions of three different salt concentrations, the nuclease P1 of the experimental group all generated degradation products, indicating enzymatic activity on the single-stranded DNA under these three different salt concentrations and good salt resistance. As shown in FIG. 4, no degradation products were observed for nuclease P1 of the experimental group under three different salt concentration conditions, indicating that P1 was not active on the double-stranded DNA under the conditions of these three different salt concentrations, and P1 could specifically degrade the single-stranded DNA in the aforementioned solution.

4. In the nanopore sequencing, a long period of continuous sequencing was usually required. Therefore, the enzyme activity of nuclease P1 placed in a high-salt environment for different times was further tested. The nuclease P1 stock solution was diluted 100 times using sequencing solution 3 to obtain sequencing solution 4. The enzyme activity of nuclease P1 placed at 30 °C for different times (0, 1, 4, 7, 16, 24 hours) was tested, with a total of five experimental groups and one control group, and each group corresponds to a time point. The specific operation was as follows: an incubation time point T was set, and the following operations were performed at 0, 1, 4, 7, 16, and 24 hours from T at 30°C: 2.7 µL of sequencing solution 4 (containing 2.7 units of activity of P1) and 5.3 µL of sequencing solution 3 were mixed, and incubated for 0, 1, 4, 7, 16, and 24 hours; 1 µL of the single-stranded DNA sequence 1 (40 ng/µL) was added to each of the six groups, and incubated at 30°C for 1 hour, and then subjected to 20% TBU polyacrylamide gel electrophoresis detection.

The results were shown in FIG. 5. It could be seen that nuclease P1 could have activity for a long time in the solution with KCl concentration of 0.47 M.

### Example 3. Interference signals found in nanopore sequencing of library

A single-channel nanopore biosensor was constructed based on phospholipid membrane (Ji, Zhouxiang, and Peixuan Guo. "Channel from bacterial virus T7 DNA packaging motor for the differentiation of peptides composed of a mixture of acidic and basic amino acids." Biomaterials 214 (2019): 119222.). Sequencing solution 3 was used, the test temperature was 30 °C, the test voltage was 0.18 V, and the test genome was *Escherichia coli* (Genbank: CP000946.1). The method was as follows: a sequencing library was constructed by ligating sequencing adapters containing *E. coli* DDA helicase with the *E. coli* genome sequences under the action of T4 DNA ligase (NEB, E6057AVIAL), followed by purification by magnetic beads for later use.

10 µL of the *E. coli* sequencing library (50 ng/µL) and 6 µL of tether sequence (1 µM, SEQ ID NO: 5) were added to 300 µL of sequencing solution 3, mixed evenly, and added in a flow cell. After incubation for 20 minutes, sequencing was started.

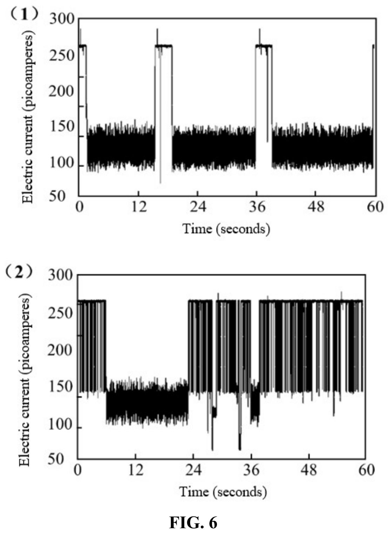

Results and analysis: The sequencing signal at the beginning of sequencing was shown in FIG. 6(1), which was the normal signal obtained by sequencing of a DNA library. After 16 hours of sequencing, the signal shown in FIG. 6(2) was obtained. Comparing FIG. 6(1) and FIG. 6(2), it could be seen that after a long period of sequencing (16 hours), the normal sequencing signals decreased, and more signals with shorter duration (i.e., non-sequencing signals) appeared. These signals could not be distinguished due to their short duration, and therefore belonged to interference signals. It was speculated that these interference signals were caused by the drawbacks of the nanopore sequencing method, specifically generated by the residual complementary single strand (i.e., non-sequencing strand) passing through the nanopore. In order to verify this speculation and reduce or eliminate these interference signals, the inventors of the present application further used nucleases to eliminate and degrade the complementary single strand.

### Example 4. Using nuclease to reduce or eliminate interference signals

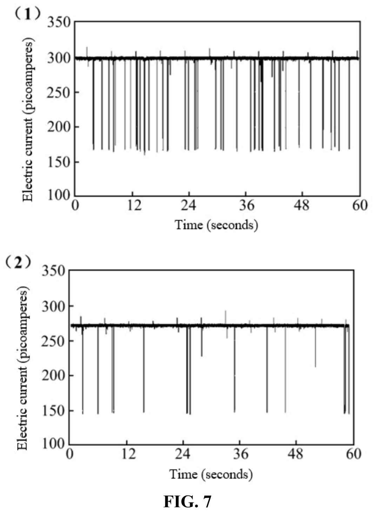

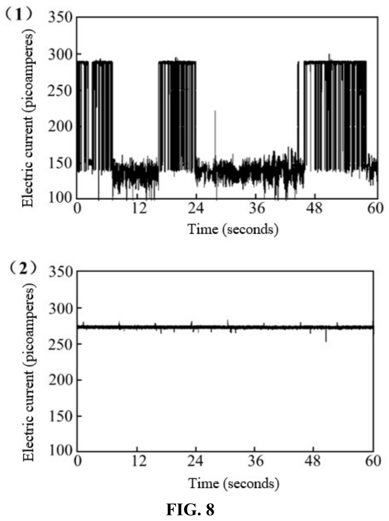

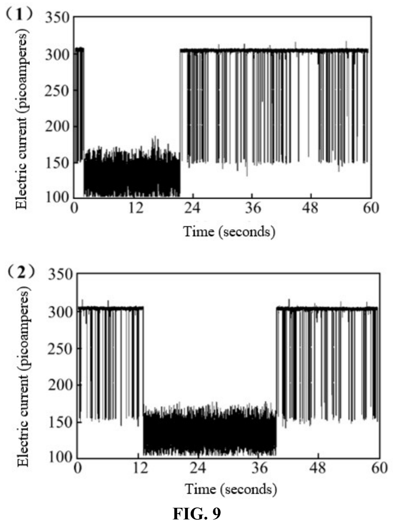

Experimental method: Interference signals were reduced or eliminated by adding an appropriate amount of nuclease to the sequencing solution. The *E. coli* sequencing library was sequenced using sequencing solution 3. A first experimental group, a second experimental group and a control group were set up with addition of different nucleases. The specific steps were carried out according to Example 3. First, normal sequencing was performed for 16 hours. The sequencing signal diagrams of the three groups were shown in FIG. 7(1), FIG. 8(1) and FIG. 9(1), respectively. Then, the sequencing was paused, and the three groups were added with nucleases according to the following operations before resuming sequencing: (a) for the first group, 300 µL of sequencing solution 6 containing nuclease P1 (10 units of activity of nuclease P1) was added to the flow cell, followed by incubation at 30 °C for 30 minutes to degrade the non-sequencing strand, and then 3 mL of sequencing solution 3 was added to wash the flow cell, and sequencing was continued for 30 minutes to obtain the sequencing signal as shown in FIG. 7(2); (b) for the second group, 300 µL of sequencing solution 5 containing nuclease P1 and nuclease T5 (100 units of activity of nuclease P1, 10 units of activity of nuclease T5) was added to the flow cell, followed by incubation at 30°C for 30 minutes to degrade the non-sequencing strand, and then 3 mL of sequencing solution 3 was injected to wash the flow cell, and sequencing was continued for 30 minutes to obtain the sequencing signal as shown in FIG. 8(2); (c) for the control group, an equal amount of sequencing solution 3 without enzyme was added, and allowed to stand at 30°C for 30 minutes, and then 3 mL of sequencing solution 3 was injected to wash the flow cell, and sequencing was continued for 30 minutes to obtain the sequencing signal as shown in FIG. 9(2).

Results and analysis: It could be seen from FIGS. 7 to 9 that the interference signal could be effectively reduced after nuclease P1 was added, and the interference signal could be effectively eliminated by adding a combination of P1 and T5 nucleases and increasing the amount of P1. In the control group without addition of nuclease, there were still a lot of interference signals, and the interference signals had not decreased, which showed that the interference signals could only be eliminated by nuclease digestion, and could not be eliminated or reduced by simply injecting the sequencing solution for replacement.

### Example 5. Evaluation of effect of nuclease on single-stranded DNA sequencing

QC DNA was used as a single-stranded DNA template (sequence 4, SEQ ID NO: 6) to evaluate the effect of enzyme on single-stranded DNA sequencing.

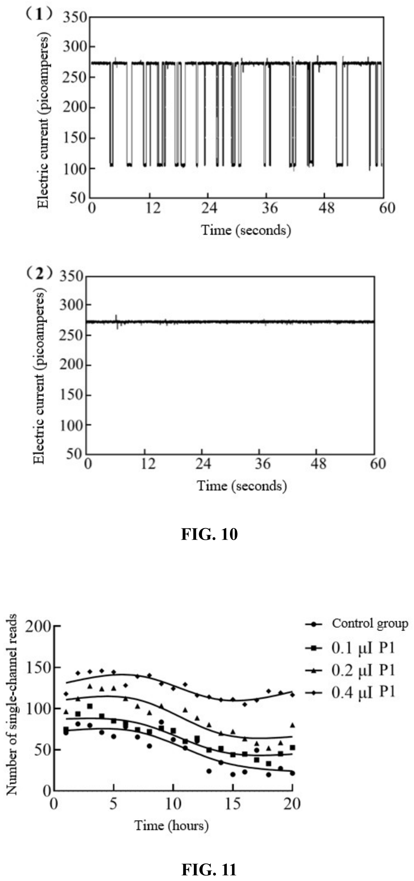

Experimental method: 3 µL of QC DNA sequencing library (20 µM) was added to sequencing solution 6 containing nuclease P1 as the experimental group; the control group was sequencing solution 3 without enzyme. After mixing evenly, the solutions were incubated at 30°C for 30 minutes, and then injected into the flow cell, followed by incubation for 20 minutes. Nanopore single-channel sequencing was performed for 30 minutes. The sequencing signals obtained in the control group and the experimental group were as shown in FIG. 10(1) and FIG. 10(2), respectively.

Results and analysis: The control group without addition of enzyme had more sequencing signals, while no sequencing signals were observed in the experimental group with addition of enzyme, which indicated that nuclease P1 could eliminate or degrade single-stranded DNA in the sequencing environment.

### Example 6. Evaluation of effect of the presence of nuclease on sequencing reads and read lengths

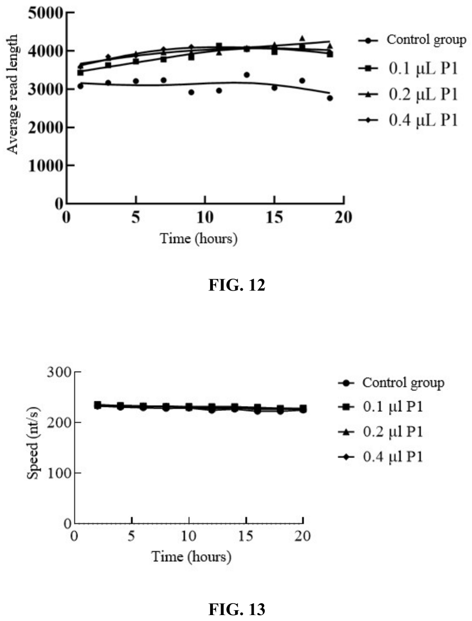

Experimental method: Sequencing solutions 6 to 8 containing different amounts of nuclease P1 were used to perform nanopore single-channel sequencing on *E. coli* sequencing library at 30°C. Sequencing solution 3 without enzyme was used as a control. The specific sequencing steps were performed in accordance with Example 3, and the experiment was repeated three times. The number of sequencing reads (read count, mean value) of a single channel per hour was counted, and the average length (read length, mean value) of sequencing reads and sequencing speed (median value) per 2-hour interval were counted. The sequencing data was processed using GraphPad Prism 8.0.2 software, in which curve fitting applying smoothing was adopted with the smoothing point value set to 4. The statistical results of sequencing reads count were shown in FIG. 11, the statistical results of sequencing read length were shown in FIG. 12, and the statistical results of sequencing speed were shown in FIG. 13.

Results and analysis: As shown in FIG. 11, compared with the control group, the addition of nuclease P1 could lead to the increase in the single-channel DNA read count; and with the increase in the amount of P1, the single-channel DNA read count increased. The reason for this could be that with the increase in the amount of nuclease P1 added, an improved efficiency of the digestion and degradation of single-stranded DNA could be achieved. In addition, there was a difference in the initial single-channel read count between the control group and the experimental group. The initial single-channel read count of the experimental group were higher than that of the control group, and the increasing trend of reads count became more pronounced with prolonged sequencing. The reason for this could be that the amount of complementary single-stranded DNA increased with prolonged sequencing, which caused an increase in "pore clogging" , and thus the effect of nuclease digestion and degradation of complementary single-stranded DNA became more significant in the later stage of sequencing, resulting in a pronounced increasing trend of read count in the later stage. In the early stage of sequencing, there were fewer complementary single-stranded DNAs, and the reason why the initial single-channel read count of the experimental group were higher than that of the control group could be due to the presence of impurity single-stranded DNA, which could be a residue in the process of library construction, or a single strand from the unwinding of double-stranded DNA during the incubation of library in the early stage of sequencing. As shown in FIG. 12, the addition of nuclease P1 could increase the average read length, but the average read length did not change with the amount of P1 added. As shown in FIG. 13, the addition of nuclease P1 had no effect on the sequencing speed. In summary, the addition of nuclease P1 could effectively increase the number of reads of a single channel and the average read length, thereby increasing sequencing throughput.

### Example 7. Effect of nuclease on gating event of pore protein during sequencing

During the sequencing process, clogging of nanopore protein channels could cause pore protein gating, resulting in detection failure. In this example, it was detected whether the addition of nuclease could reduce the frequency of pore protein gating event during sequencing.

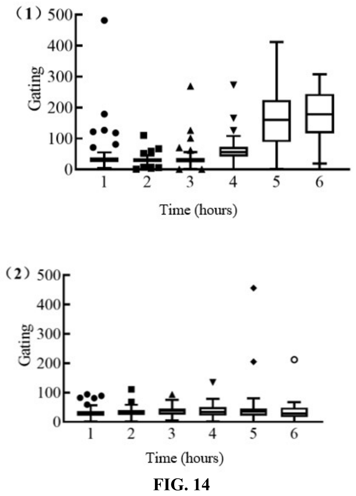

Experimental method: In the experimental group, sequencing solution 6 was used to perform nanopore single-channel sequencing on the *E. coli* sequencing library at 32°C; and in the control group, solution 3 was used without enzyme. The specific sequencing steps were carried out with reference to Example 3. The gating frequencies of pore protein in different channels were recorded, and more than 40 channels were counted to calculate the mean value. The results were shown in FIG. 14.

Results and analysis: The gating frequency could represent the clogging of nanopore channel to a certain extent. As the amount of non-sequencing DNA single strands generated during the sequencing process increased, the clogging phenomenon would occur, which would increase the gating frequency. As shown in FIG. 14, the gating frequencies of the experimental group and the control group were similar in the first 3 hours of sequencing. Starting from the 4^{th} hour of sequencing, the gating frequency of the control group increased significantly, while the experimental group remained at a relatively low level. Therefore, the experimental results of this example proved that the addition of nuclease P1 could effectively reduce the gating frequency.

### Example 8. Exploring time point for adding nuclease

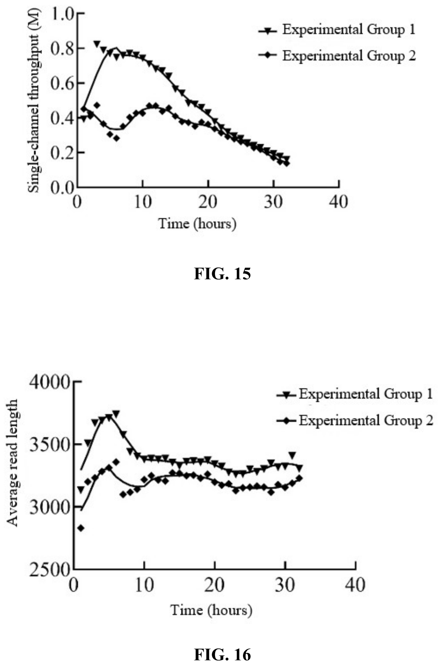

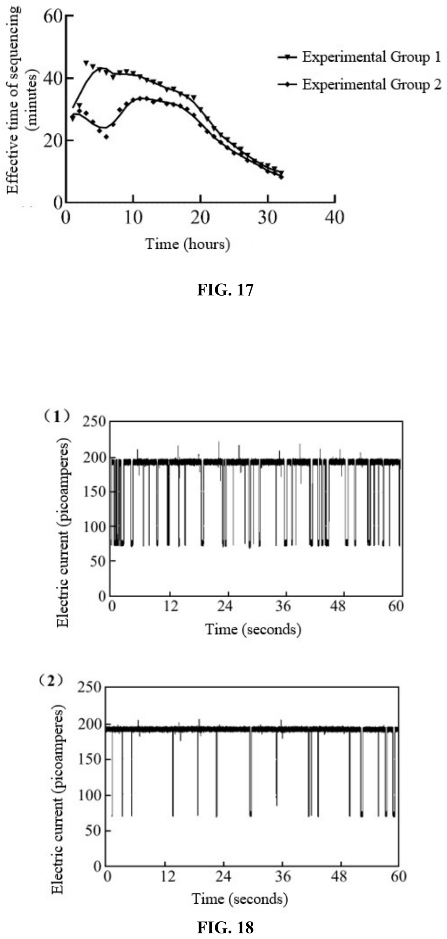

Experimental method: Sequencing solution 3 was used, the test temperature was 34 °C, the test voltage was 0.18 V, and the test genome was *Bacillus subtilis* (Genbank: NC000964.3). The specific steps were as follows: the *Bacillus subtilis* sequencing library was constructed according to the method in Example 3, 1.5 µg of sequencing library and 3.5 µL of tether (10 M) were added to 400 µL of sequencing solution 3 in an EP tube, and mixed well; after incubation for 30 minutes, the following two groups of experiments were performed: (1) Experimental Group 1: enzyme was added at the initial 0 hour, specifically, 0.75 µL of nuclease P1 (containing 75 units of activity) was added to the above sequencing system, which was then added to the flow cell and incubated for 20 minutes to start sequencing. After 6 hours of sequencing, 1.0 µg of sequencing library and 1.0 µL of tether were added to 400 µL of sequencing solution 3 in the EP tube, and mixed well; after incubation for 30 minutes, 0.75 µL of nuclease P1 was added, and then injected into the flow cell to replace the sequencing solution, and sequencing was continued for 26 hours; (2) Experimental Group 2: enzyme was added after 6 hours of sequencing (i.e., the sequencing solution with enzyme was added to replace the sequencing solution without enzyme in the flow cell after 6 hours of sequencing). Specifically, the above sequencing system was directly added to the flow cell, and incubated for 20 minutes to start sequencing. After 6 hours of sequencing, 1.0 µg of sequencing library and 1.0 µL of tether (10 M) were added to 400 µL of sequencing solution 3 in the EP tube and mixed evenly. After incubation for 30 minutes, 0.75 µL of enzyme P1 was added and injected into the flow cell to replace the sequencing solution,, and the sequencing was continued for 26 hours. The single channel throughput per hour (as shown in FIG. 15), average read length (as shown in FIG. 16), and effective sequencing time (as shown in FIG. 17) were calculated.

Results and analysis: As shown in FIG. 15, the single channel throughput of Experimental Group 1 was greater than that of Experimental Group 2 in the first 6 hours of sequencing. The reason for the increase in throughput could be the increased effective sequencing time (as shown in FIG. 17), as the addition of nuclease alleviated the "pore clogging" caused by interference single-stranded DNA. In addition, the single channel throughput of Experimental Group 2 showed a slight downward trend in the first 6 hours, but the throughput increased after the addition of nuclease P1. As shown in FIG. 16, the average read length of the sequenced DNA in Experimental Group 1 was slightly longer than that in Experimental Group 2. From the above results, it could be seen that the addition of nuclease P1 at the beginning of sequencing had greater advantages in single channel throughput, average read length and effective sequencing time.

### Example 9. Evaluation of effect of presence of nuclease HL.exo on single-stranded DNA sequencing

The effect of the presence of nuclease HL.exo on single-stranded DNA sequencing was evaluated using a single-stranded DNA template (sequence 4, SEQ ID NO: 6).

Experimental method: 4 µL of QC DNA sequencing library (20 µM) was taken and added to 300 µL of sequencing solution 9 containing nuclease HL.exo (10.32 units of activity of nuclease HL.exo) as the experimental group; the control group was sequencing solution 2 without enzyme. After mixing evenly, the solutions were incubated at 30°C for 20 minutes, and then injected into the flow cell, followed by incubation for 10 minutes. Nanopore single-channel sequencing was then performed for 10 minutes. The sequencing signals obtained in the control group and the experimental group were shown in FIG. 18 (1) and FIG. 18 (2), respectively.

Results and analysis: The control group without addition of enzyme had more sequencing signals, while the sequencing signals in the experimental group with nuclease HL.exo decreased, indicating that the nuclease HL.exo could degrade single-stranded DNA in the sequencing environment.

Although the specific embodiments of the present invention have been described in detail, those skilled in the art will understand that various modifications and changes can be made to the details based on all the teachings that have been disclosed, and these changes are within the scope of protection of the present invention. The entirety of the present invention is given by the attached claims and any equivalents thereof.

## Claims

1. A method for nucleic acid sequencing, comprising performing nanopore sequencing on a polynucleotide to be tested under a condition comprising a single-strand-specific nuclease.

2. The method according to claim 1, wherein the condition comprising the single-strand-specific nuclease comprises adding the single-strand-specific nuclease to a sequencing system;
preferably, the single-strand-specific nuclease is added to the sequencing system before the start of, during and/or after sequencing;
preferably, incubation is further comprised after the single-strand-specific nuclease is added to the sequencing system.

3. The method according to claim 1 or 2, wherein the nanopore sequencing comprises:
- providing a condition in a sequencing system comprising the polynucleotide to be tested and a sequencing solution so as to allow a single-stranded analyte of the polynucleotide to be tested to enter the nanopore; and
- obtaining one or more measurement values while moving the single-stranded analyte with respect to the nanopore, wherein the measurement values represent the sequence information of the polynucleotide to be tested.

4. The method according to any one of claims 1 to 3, wherein the single-strand-specific nuclease is added to a sequencing system before the start of sequencing;
preferably, the method comprises: providing a condition in a sequencing system with the addition of the single-strand-specific nuclease so as to allow a single-stranded analyte of the polynucleotide to be tested to enter the nanopore to perform sequencing.

5. The method according to any one of claims 1 to 3, wherein the single-strand-specific nuclease is added to a sequencing system during sequencing;
preferably, the method comprises:
- providing a condition in a sequencing system without the addition of the single-strand-specific nuclease so as to allow the single-stranded analyte of the polynucleotide to be tested to enter the nanopore to start sequencing;
- adding the single-strand-specific nuclease to the sequencing system once or more times.

6. The method according to claim 5, wherein the method comprises:
- providing a condition in the sequencing system without the addition of the single-strand-specific nuclease so as to allow the single-stranded analyte of the polynucleotide to be tested to enter the nanopore to start sequencing;
- pausing sequencing and adding the single-strand-specific nuclease to the sequencing system;
- resuming sequencing;
preferably, the steps of "pausing sequencing and adding the single-strand-specific nuclease to the sequencing system; resuming sequencing" are repeated once or more times until sequencing is completed.

7. The method according to any one of claims 1 to 3, wherein the single-strand-specific nuclease is added to a sequencing system after sequencing is completed;
preferably, the method further comprises a washing step (e.g., using a sequencing solution);
preferably, the method further comprises proceeding to a next round of sequencing.

8. The method according to any one of claims 1 to 7, wherein the single-strand-specific nuclease is selected from an endonuclease or an exonuclease;
preferably, the single-strand-specific nuclease is selected from nuclease P1, nuclease T5, nuclease RecJ_{f}, nuclease HL.exo, nuclease Lambda, nuclease MBN, nuclease T7, nuclease S1, nuclease BAL 31, nuclease Mung bean, or any combination thereof;
preferably, the single-strand-specific nuclease is selected from nuclease P1, nuclease T5, nuclease RecJ_{f}, nuclease HL.exo, or any combination thereof.

9. The method according to any one of claims 1 to 8, wherein the single-strand-specific nuclease has a concentration of at least 0.01 U/µL, such as 0.01 to 100 U/µL;
preferably, the single-strand-specific nuclease has a concentration of 0.01 to 50 U/µL, such as 0.01 to 20 U/µL, 0.01 to 10 U/µL, 0.01 to 5 U/µL, or 0.01 to 1 U/µL.

10. The method according to any one of claims 1 to 9, wherein the condition that allows the single-stranded analyte of the polynucleotide to be tested to enter the nanopore comprises: contacting the polynucleotide to be tested with a polynucleotide binding protein and a nanopore in a sequencing solution, wherein the polynucleotide binding protein controls the single-stranded analyte of the polynucleotide to be tested to move through the nanopore.

11. The method according to claim 10, wherein the polynucleotide binding protein is selected from a nucleic acid helicase, such as a DNA helicase or an RNA helicase;
preferably, the polynucleotide binding protein is selected from Dda, UvrD, Rep, RecQ, PcrA, eIF4A, NS3, Rep, gp41 or T7gp4.

12. The method according to claim 10 or 11, wherein the condition further comprises: providing a potential difference across the nanopore to allow the single-stranded analyte to enter the nanopore.

13. The method according to any one of claims 1 to 12, wherein the sequencing system comprises a sequencing solution, and the sequencing solution comprises a charge carrier, such as a salt (e.g., potassium chloride (KCl), sodium chloride (NaCl), cesium chloride (CsCl), or a mixture of potassium ferrocyanide and potassium ferricyanide);
preferably, the sequencing solution further comprises a buffer, such as a HEPES buffer, a phosphate buffer or a Tris-HCl buffer;
preferably, the sequencing solution further comprises a free nucleotide (e.g., AMP, TMP, GMP, CMP, UMP, dAMP, dTMP, dGMP or dCMP, such as adenosine triphosphate (ATP)) and/or an enzyme cofactor (e.g., a divalent metal cation, such as Mg²⁺, Mn²⁺, Ca²⁺ or Co²⁺);
preferably, the sequencing solution comprises potassium chloride (KCl), HEPES buffer, ATP and Mg²⁺.

14. The method according to any one of claims 1 to 13, wherein the polynucleotide to be tested is a double-stranded polynucleotide.

15. The method according to claim 14, wherein the double-stranded polynucleotide comprises at least one single-stranded overhang (e.g., 5'-end overhang and/or 3'-end overhang), and the single-stranded overhang comprises a leader sequence, and the leader sequence leads a nucleic acid strand attached thereto into the nanopore.

16. The method according to any one of claims 1 to 15, wherein the sequencing system further comprises one or more tethers, the tether comprises a capture sequence having sequence complementarity with a portion of the polynucleotide to be tested, and the polynucleotide to be tested is coupled to an outer rim or vicinity of the nanopore by hybridization of the capture sequence with the complementary region in the polynucleotide to be tested.

17. The method according to any one of claims 1 to 16, comprising the following steps:
(1) providing a double-stranded polynucleotide to be tested comprising a single-stranded overhang, wherein the single-stranded overhang is linked with a polynucleotide binding protein;
(2) contacting the double-stranded polynucleotide to be tested of step (1) with a nanopore in a sequencing solution comprising the single-strand-specific nuclease, wherein the polynucleotide binding protein separates the two strands of the double-stranded polynucleotide to provide a single-stranded polynucleotide (i.e., a single-stranded analyte), and controls the movement of the single-stranded analyte through the pore; and,
(3) obtaining one or more measurement values while moving the single-stranded analyte with respect to the nanopore, wherein the measurement values can be used to represent the sequence information of the polynucleotide to be tested.

18. The method according to claim 17, wherein step (2) comprises: providing the double-stranded polynucleotide to be tested of step (1), the single-strand-specific nuclease and a tether in a sequencing solution, and incubating, and then contacting the incubated solution with the nanopore.

19. The method according to any one of claims 1 to 18, wherein the nanopore is disposed in a membrane;
preferably, the membrane is an amphiphilic layer, such as a lipid bilayer (e.g., a phospholipid bilayer) or a polymer membrane (e.g., a di-block, tri-block).

20. A kit, comprising a single-strand-specific nuclease;
preferably, the kit is used for nanopore sequencing;
preferably, the single-strand-specific nuclease is selected from an endonuclease or an exonuclease;
preferably, the single-strand-specific nuclease is selected from nuclease P1, nuclease T5, nuclease RecJ_{f}, nuclease HL.exo, nuclease Lambda, nuclease MBN, nuclease T7, nuclease S1, nuclease BAL 31, nuclease Mung bean, or any combination thereof; preferably, the single-strand-specific nuclease is selected from nuclease P1, nuclease T5, nuclease RecJ_{f}, nuclease HL.exo, or any combination thereof.

21. The kit according to claim 20, further comprising a reagent for nanopore sequencing;
preferably, the reagent for nanopore sequencing comprises one or more selected from the following: a nanopore or subunit thereof, a membrane (e.g., one as defined in claim 19), a polynucleotide binding protein (e.g., one as defined in claim 11), a sequencing solution (e.g., one as defined in claim 13), a tether (e.g., one as defined in claim 16).

22. The kit according to claim 20 or 21, wherein the single-strand-specific nuclease has a concentration of at least 0.01 U/µL, such as 0.01-100 U/µL;
preferably, the single-strand-specific nuclease has a concentration of 0.01-50 U/µL, such as 0.01-20 U/µL, 0.01-10 U/µL, 0.01-5 U/µL or 0.01-1 U/µL.

23. Use of the kit according to any one of claims 20 to 22 for nanopore sequencing.

24. Use of a single-strand-specific nuclease for nanopore sequencing.
